# EUROPEAN PATENT APPLICATION

(11) **EP 4 806 993 A1**
(43) Date of publication of application: **16.09.2026**
(21) Application number: 24889157.4
(22) Date of filing: 07.11.2024
(51) Int. Cl.: C12N 9/18, C12N 15/70, C12N 15/75, A61K 38/46, A61K 31/225, A61K 35/74, A61K 35/742, A61P 1/00, A61P 29/00

(54) **NOVEL BACILLUS SUBTILIS BM107 STRAIN AND USE THEREOF**

(30) Priority: 08.11.2023 KR 20230153948; 04.09.2024 KR 20240120395; 04.09.2024 KR 20240120397
(71) Applicant: Biome Inc., Seodaemun-gu Seoul 03722 (KR)
(72) Inventor: YOON, Sang Sun, Seoul 03477 (KR); YOON, Mi Young, Seoul 02863 (KR)
(74) Representative: Dragsted Partners A/S
(86) International application number: PCT/KR2024/017553
(87) International publication number: WO 2025/100971

(57) **Abstract**

The present disclosure relates to: Bacillus subtilis comprising a gene encoding a novel esterase; and a use thereof. The present disclosure provides a novel Bacillus subtilis which can efficiently enhance the production of butyrate by breaking down tributyrin in the body or intestine, and exhibits therapeutic and preventive effects on inflammatory bowel disease on the basis thereof.

## Description

### Technical Field

The present disclosure relates to Bacillus subtilis containing a gene encoding a novel esterase and its uses.

### Background Art

Butyrate or butyric acid, a type of short-chain fatty acid, can be produced by the fermentation of dietary fibers by butyrate-producing bacteria present in the intestines of humans or animals. Recent studies have shown that butyrate exhibits various physiological effects, such as anti-inflammatory actions and the prevention of colorectal cancer, and that it can help prevent or improve various diseases.

More specifically, butyrate serves as the primary energy source for colon cells and can maintain the integrity of the intestinal mucosal layer, preventing leaky gut. Furthermore, it has been revealed that butyrate has the effect of reducing inflammatory responses, and some studies have also reported effects related to a reduced risk of colorectal cancer. Moreover, studies indicate that butyrate positively affects brain health and cognitive function by promoting nerve cell growth and reducing brain inflammation.

For these reasons, various attempts are being made to utilize butyrate for the prevention and treatment of diseases or for health enhancement. On the other hand, while butyrate is produced by the fermentation of dietary fibers, the intake of dietary fibers alone has limitations in producing sufficient amounts of butyrate. Additionally, due to its own unpleasant odor, it may be difficult to add butyrate directly to supplements, pharmaceuticals, or foods, and managing its concentration is challenging because butyrate is a volatile substance. As a result, various treatments are being conducted to increase butyrate levels in the intestines of humans and animals.

Therefore, there is a continuous demand for the development of a new butyrate-enhancement system that effectively enhances butyrate levels in the intestines.

The background technology is provided to facilitate better understanding of this disclosure. The subject matter described in the background technology should not be construed as admitted prior art.

### Disclosure of Invention

### Technical Problem

As a method to enhance butyrate levels in the intestines, a butyrate-producing intestinal enhancer using *Clostridium butylicum* as the active ingredient has been proposed.

Such probiotic compositions can further enhance intestinal butyrate levels, but it may be difficult to set a target intestinal butyrate concentration, and the colonization rate of Clostridium butyricum may vary among individuals. In other words, the efficiency of butyrate production via enhancement based on butyrate-producing bacteria may vary among individuals. Furthermore, in producing the enhancer, Clostridium butyricum may require more stringent culture conditions than rapidly dividing or anaerobic strains such as Escherichia coli. Moreover, when producing recombinant strains with similar mechanisms, Escherichia coli strains are used, and in such cases, stability issues arise, including the lipopolysaccharide problem of Gram-negative Escherichia coli.

The inventors of the present disclosure aimed to develop a new intestinal butyrate level enhancement system to address the limitations of the conventional intestinal butyrate production enhancers mentioned above.

Meanwhile, the inventors of the present disclosure focused on *Bacillus subtilis*, which produces butyrate using tributyrin as a substrate.

More specifically, *Bacillus subtilis* is a gram-positive bacterium lacking lipopolysaccharides, a safe bacterium with a history of dietary use and a proven track record of development into health-functional foods and pharmaceuticals.

Accordingly, the inventors of the present disclosure have identified bacteria capable of producing butyrate from healthy individuals, among which they discovered a strain of *Bacillus subtilis* that does not show toxicity to the host and has improved butyrate production efficiency. Furthermore, by identifying the *Bacillus subtilis* strain they discovered, they found a specific factor that enhances butyrate production efficiency: the estB gene, which increases esterase activity.

Ultimately, the inventors of the present disclosure expect that when Bacillus subtilis containing the estB gene, which can enhance the aforementioned esterase activity, is used in the intestine, it can effectively enhance butyrate production in the intestinal environment and prevent or treat intestinal diseases by producing butyrate.

Therefore, the task to be solved by the present disclosure is to provide an estB gene encoding an esterase derived from *Bacillus subtilis* that can enhance the intrinsic activity of the esterase in microorganisms.

Additionally, another task addressed by the present disclosure is to provide a composition for the prevention or treatment of inflammatory bowel diseases that can be effectively applied to the treatment or prevention of various inflammatory bowel diseases by further enhancing butyrate production efficiency through the inclusion of butyrate along with the aforementioned Bacillus subtilis.

The tasks of the present disclosure are not limited to the tasks mentioned above, and other tasks not mentioned will be clearly understood by those skilled in the art from the description below.

### Solution to Problem

To solve the aforementioned tasks, the present disclosure provides a gene indicated by the nucleotide sequence of SEQ ID NO. 1, which encodes an esterase derived from *Bacillus subtilis*.

To solve another task as described above, it provides an esterase indicated by the amino acid sequence of SEQ ID NO. 2.

According to another feature of the present disclosure, the esterase may be capable of producing butyrate by degrading the substrate tributyrin, but is not limited to this.

According to another feature of the present disclosure, the esterase may be expressed from a gene indicated by the nucleotide sequence of SEQ ID NO. 1 derived from *Bacillus subtilis*, but is not limited to this.

To solve another task as described above, it provides a recombinant vector containing a gene encoding an esterase, as indicated by nucleotide sequence 1.

To solve another task as described above, it provides a host cell transformed with the aforementioned recombinant vector.

According to another feature of the present disclosure, the host cell may produce an esterase indicated by the amino acid sequence of SEQ ID NO. 2, but is not limited to this.

According to another feature of the present disclosure, the host cell may have increased activity of the esterase compared to the wild type, but is not limited to this.

According to another feature of the present disclosure, the host cell may be *Escherichia coli* or *Bacillus subtilis*, but is not limited to this.

According to another feature of the present disclosure, the *Bacillus subtilis* may be *Bacillus subtilis* BM107 deposited under the Accession Number KCTC 15208BP, but is not limited to this.

To solve another task as described above, it provides *Bacillus subtilis* BM107 deposited under the Accession Number KCTC 15208BP, which has excellent butyrate production ability.

According to another feature of the present disclosure, the Bacillus subtilis BM107 may be an overexpressed gene indicated by the nucleotide sequence of SEQ ID NO. 1, but is not limited to this.

According to another feature of the present disclosure, the Bacillus subtilis BM107 may produce butyrate by degrading tributyrin, but is not limited to this.

According to another feature of the present disclosure, the Bacillus subtilis BM107 may have negative activity of β-Hemolysin, but is not limited to this.

According to another feature of the present disclosure, the Bacillus subtilis BM107 may not have resistance to at least one of the antibiotics vancomycin, gentamicin, kanamycin, erythromycin, clindamycin, tetracycline, chloramphenicol, but is not limited to this.

According to another feature of the present disclosure, Bacillus subtilis BM107 may have a tributyrin-degrading rate more than 1.2 times that of Bacillus subtilis lacking the gene indicated by SEQ ID NO. 1, but this is not limited to that.

According to another feature of the present disclosure, the Bacillus subtilis BM107 may have a tributyrin-degrading rate more than 17 times that of *Lactobacillus sp.*, but this is not limited to that.

According to another feature of the present disclosure, Bacillus subtilis BM107 may have a tributyrin-degrading rate more than 34.5 times that of *Weissella sp*.

According to another feature of the present disclosure, a recombinant vector containing the gene indicated by the SEQ ID NO. 1 and host cells transformed with it, along with a pharmaceutical composition for the prevention or treatment of inflammatory bowel disease containing tributyrin, is provided.

According to another feature of the present disclosure, the inflammatory bowel diseases may include ulcerative colitis, Crohn's disease, collagenous colitis, lymphocytic colitis, ischemic colitis, transitional colitis, and Behçet's syndrome, but this is not limited to that, and may include various intestinal diseases that involve inflammatory responses in the intestines.

On the other hand, according to another feature of the present disclosure, the composition for the prevention or treatment of inflammatory bowel disease according to one embodiment of the present disclosure can be applied to diseases that can be improved through enhanced butyrate production, as well as inflammatory bowel diseases. For example, the composition for the prevention or treatment of inflammatory bowel disease according to one embodiment of the present disclosure can be applied to diseases such as cancer, obesity, diabetes, metabolic syndrome, cardiovascular diseases, dementia, depression, brain diseases, and osteoporosis. In addition, the composition for the prevention or treatment of inflammatory bowel disease according to one embodiment of the present disclosure is not limited to the aforementioned diseases and can be expected to have antioxidant effects within the individual.

According to another feature of the present disclosure, the composition can be formulated in a rectally administrated form or an orally ingestible form, but this is not limited to that, and the composition for the prevention or treatment of inflammatory bowel disease according to one embodiment of the present disclosure can include various administration methods that allow it to be delivered to the host's body to degrade intestinal tributyrin. However, most preferably, it may be in a rectally administered form or an orally ingestible form.

To address another problem as described above, a method is provided comprising the step of transforming host cells with a recombinant vector containing the gene indicated by SEQ ID NO. 1 derived from Bacillus subtilis, and the step of overexpressing the gene indicated by SEQ ID NO. 1, which enhances esterase activity.

### Advantageous Effects of Invention

The present disclosure enhances the butyrate production in host cells and has therapeutic and preventive effects on inflammatory bowel diseases based on this.

More specifically, the present disclosure can efficiently enhance butyrate production by degrading tributyrin in the body or intestines, using a novel *Bacillus subtilis*. Furthermore, the present disclosure can further enhance butyrate production by providing tributyrin in a prodrug form along with Bacillus subtilis.

In particular, after inducing butyrate production in the intestines, it can be directly delivered there, thereby overcoming the limitations of conventional butyrate, which are difficult to ingest and administer due to issues such as odor and volatility.

Furthermore, by controlling the level of the substrate tributyrin, it is easier to set the target intestinal butyrate concentration, thereby improving intestinal butyrate production efficiency compared to conventional butyrate-producing bacteria.

The effects according to the present disclosure are not limited by the examples illustrated above, and more various effects are included in this specification.

### Brief Description of Drawings

Fig. 1 is a flowchart of the identification process for Bacillus subtilis strains containing the esterase-encoding gene according to one embodiment of the present disclosure.
Fig. 2 is a schematic diagram of the tributyrin-degrading process of *Bacillus subtilis* containing the esterase-encoding gene according to one embodiment of the present disclosure.
Fig. 3 shows the results of hemolysis confirmation for the bacterial strain, Bacillus subtilis, containing the esterase-encoding gene according to one embodiment of the present disclosure.
Fig. 4 shows the evaluation results for the tributyrin-degrading ability of the bacterial strain containing the esterase-encoding gene according to one embodiment of the present disclosure.
Fig. 5a shows the result of strain selection based on the tributyrin-degrading ability of Bacillus subtilis containing the esterase-encoding gene according to an embodiment of the present disclosure.
Fig. 5b shows the PCR results of Bacillus subtilis containing the esterase-encoding gene according to one embodiment of the present disclosure.
Figs. 6a and 6b show transformed Escherichia coli containing an esterase-coding gene according to one embodiment of the present invention, along with the results of its tributyrin degradation ability.
Fig. 6c shows the result of Sanger sequencing analysis of the transformed Escherichia coli colony containing the esterase-encoding gene according to an embodiment of the present disclosure.
Fig. 6d shows a clear zone around a transformed Escherichia coli colony containing the esterase-encoding gene according to an embodiment of the present disclosure.
Figs. 7a and 7b show the experimental results regarding the antibiotic resistance of bacterial strains containing the esterase-encoding gene according to an embodiment of the present disclosure according to an embodiment of the present disclosure.
Fig. 8a shows the intestinal length results after treatment with the bacterial strain, Bacillus subtilis, containing an esterase-coding gene according to one embodiment of the present invention, and a prodrug composition for the prevention or treatment of inflammatory bowel disease containing the same.
Fig. 8b shows the result of H&E stained images of colon tissue.
Fig. 8c shows intestinal butyrate concentration results after treatment with the bacterial strain Bacillus subtilis containing an esterase-coding gene according to one embodiment of the present invention, and a prodrug composition for the prevention or treatment of inflammatory bowel disease containing the same.

### Best Mode for Carrying out the Invention

On the other hand, when described as a component (e.g., a first component) is "directly coupled" or "directly connected" to another component (e.g., a second component), it may be understood that no any other component (e.g., a third component) exists between said component and said other component.

The expression "configured to" used herein may be interchangeable depending on the context, for example, "suitable for," "having the capacity to," "designed to," "adapted to," "made to," or "capable of." The term "configured to" does not necessarily mean only "specifically designed to" in a hardware sense. Instead, in some contexts, the phrase "a device configured to" may mean that the apparatus, together with other apparatuses or components, is "capable of" an operation. For example, the phrase "processor configured to perform A, B, and C" may refer to a dedicated processor for performing those operations (e.g.: an embedded processor), or a generic-purpose processor (e.g.: CPU or application processor) that may perform those operations by running one or more software programs stored in a memory device.

The terms used in this document are merely used to describe specific embodiments and are not intended to limit the scope of other embodiments. Singular expressions may include plural expressions unless the context clearly indicates otherwise. The terms used here, including technical or scientific terms, may have the same meaning as generally understood by those skilled in the art in the technical field described in this document. Terms defined in general dictionaries among the terms used in this document may be interpreted with the same or similar meaning in the context of the relevant technology, and unless explicitly defined in this document, they are not interpreted in an ideal or overly formal sense. In some cases, even if a term is defined in this document, it may not be interpreted as excluding the embodiments of this document.

Each feature of the various embodiments of this disclosure may be partially or wholly combined or integrated with each other, and as those skilled in the art would easily understand, various technical interconnections and operations are possible. Each embodiment may be independently implemented with respect to each other or may be implemented together in a related manner.

Advantages and features of the present disclosure and methods of achieving the same will become apparent with reference to the embodiments described below in detail, along with the accompanying drawings. However, this disclosure is not limited to the embodiments disclosed below, but will be implemented in various different forms, and these embodiments are provided merely to make this disclosure complete and to fully inform those skilled in the art to which this disclosure pertains of the scope of the disclosure, this disclosure is defined only by the scope of the claims.

Hereinafter, for the clarity of the description, the terms used herein will be explained.

The term "or" used herein means "and/or" unless otherwise stated.

The term "about" used herein refers to the ordinary range of error for each value that is readily known to those skilled in the art. The reference to an "about" value or parameter in this specification includes embodiments concerning that value or parameter itself. Furthermore, the term "about" indicates a range of values that fall within 10% in either direction (above or below) of the referenced value unless otherwise stated or made clear from the context.

As used herein, the term "patient or individual" refers interchangeably to any single animal requiring treatment, more preferably a human or a mammal other than a human (including such non-human animals, e.g., cats, dogs, horses, rabbits, zoo animals, cattle, pigs, sheep, and non-human primates). The patient referred to in various embodiments of this specification may be a human.

The term "esterase" used herein refers to an enzyme that hydrolyzes ester bonds and can be interchangeably used with esterase, ester hydrolase, etc. Preferably, the esterase in this specification may be tributyrin esterase. More specifically, tributyrin esterase is an enzyme that hydrolyzes the ester bond of tributyrin using tributyrin as a substrate, and the hydrolysis products can yield butyric acid and glycerol.

The term "butyric acid" as used herein is one of the short-chain fatty acids and can also be understood as butyrate. In this case, butyric acid may be a natural in vivo product produced mainly in the large intestine or rumen by microbial fermentation of carbohydrates (cellulose, hemicellulose, starch). On the other hand, butyric acid can serve as an important fuel for intestinal epithelial cells, contributing to gut health and potentially reducing gut inflammation, which may be effective against various intestinal diseases, including inflammatory bowel disease. Furthermore, butyric acid may have anticancer, neuroprotective, immune-enhancing, anti-inflammatory, and weight-reducing and diabetes-preventing effects.

The term "intrinsic activity" used herein may refer to the active state that microorganisms naturally possess. For example, "enhanced compared to intrinsic activity, Escherichia coli" may refer to Escherichia coli with hydrolytic activity improved relative to the esterase activity originally possessed by Escherichia coli.

The term "homology" used herein may indicate the degree of similarity with the nucleotide sequence or amino acid sequence disclosed herein. Specifically, homology comparisons can be expressed as a percentage (%) of homology between two or more sequences. For example, through homology comparison, a polypeptide comprising an amino acid sequence that is more than 80% identical to the esterase amino acid sequence, according to various embodiments of the present disclosure, can be considered to retain the esterase's biological activity substantially.

For example, a gene consisting of a nucleotide sequence that is 80% or more identical to the nucleotide sequence encoding an esterase indicated by the nucleotide sequence of SEQ ID NO. 1 through homology comparison can be considered to be capable of encoding an esterase having the same activity as the biological activity of the esterase according to one embodiment of the present disclosure.

Additionally, the esterase may include polypeptides that have at least 80%, 90%, 95%, 96%, 97%, 98%, or 99% homology with the amino acid sequence of SEQ ID NO. 2, but is not limited to this. Moreover, if the amino acid sequence has such homology and exhibits corresponding efficacy to the esterase, it is evident that auxiliary proteins with some sequences having deletions, modifications, substitutions, or additional amino acid sequences are also included within the scope of the present disclosure.

Additionally, the polypeptides provided in the present disclosure may be further modified by chemical or post-translational modifications to enhance the esterase's intrinsic activity. Such modifications include pegylation, albuminization, glycosylation, farnesylation, carboxylation, hydroxylation, desialylation, carbamylation, sulfation, phosphorylation, and other polypeptide modifications known in the art, but are not limited to these.

The term "expression" used herein may refer to the expression or production of a polypeptide from a gene encoding for the esterase in the transformed host cell.

Isolation and purification refer to the process of isolating and purifying the polypeptide as the active ingredient. For example, the process of isolating and purifying the polypeptide according to one embodiment of the present disclosure can use methods known in the art, without limitation, and preferably utilize ion exchange chromatography. At this time, the polypeptide may be an esterase according to various embodiments of the present disclosure.

The term "pharmaceutical composition for the prevention or treatment of intestinal diseases" used herein may refer to a pharmaceutical composition that has a therapeutic effect on intestinal diseases.

In this case, if the pharmaceutical composition is formulated as a liquid solution, it may be diluted by a pharmaceutically acceptable carrier. More specifically, a pharmaceutically acceptable carrier may refer to a carrier or diluent that does not stimulate the organism and does not inhibit the biological activity and properties of the administered compound. For example, acceptable pharmaceutical carriers in a pharmaceutical composition formulated as a liquid solution include sterile and biocompatible substances such as saline, sterile water, Ringer's solution, buffered saline, albumin injection solution, dextrose solution, maltodextrin solution, glycerol, ethanol, and mixtures containing one or more of these components. Additionally, other conventional additives such as antioxidants, buffers, and bacteriostatic agents may be added as needed. Furthermore, diluents, dispersants, surfactants, binders, and lubricants may be additionally added, enabling the composition for the prevention or treatment of intestinal diseases to be formulated into injectable formulations such as aqueous solutions, suspensions, and emulsions, as well as pills, capsules, granules, or tablets.

The composition for the prevention or treatment of intestinal diseases can be administered orally or non-orally, and may also be applied or sprayed to the site of the disease. The composition for the prevention or treatment of intestinal diseases may also be administered non-oral routes, such as intravenous, intraperitoneal, intramuscular, subcutaneous, or local administration.

Suitable application, spraying, and dosage of a composition for the prevention or treatment of intestinal diseases may vary depending on factors such as the method of formulation, the administration mode, the age, body weight, sex of the target animal or patient, severity of disease symptoms, dietary, time of administration, route of administration, excretion rate, and response sensitivity. Additionally, a skilled physician or veterinarian can easily determine and prescribe the dosage of the composition for the prevention or treatment of intestinal diseases that is effective for the intended treatment.

Oral dosage forms containing active ingredients for the prevention or treatment of intestinal diseases may include tablets, troches, lozenges, aqueous or oily suspensions, powdered or granulated formulations, emulsions, hard or soft capsules, syrups, or elixirs. At this time, to formulate into formulations such as tablets and capsules, binders such as lactose, saccharose, sorbitol, mannitol, starch, amylopectin, cellulose, or gelatin, excipients such as dicalcium phosphate, disintegrants such as corn starch or sweet potato starch, lubricants such as magnesium stearate, calcium stearate, sodium stearyl fumarate, or polyethylene glycol wax may be included, and in the case of capsule formulations, in addition to the aforementioned substances, a liquid carrier such as fatty oil may be further included.

The term "intestinal disease" as used herein may refer to at least one of inflammatory bowel disease, Clostridium difficile infection, Crohn's disease, irritable bowel syndrome, ulcerative colitis, Behçet's disease, tuberculous enteritis, and ischemic bowel disease. Preferably, in this specification, intestinal disease may be inflammatory bowel disease, but it is not limited to this. Furthermore, such inflammatory bowel diseases may include at least one of ulcerative colitis, Crohn's disease, collagenous colitis, lymphocytic colitis, ischemic colitis, transitional colitis, and Behçet's syndrome.

The term "prodrugs" as used herein refers to drugs that have been chemically modified to control their physical and chemical properties, which do not exhibit physiological activity by themselves but can be converted into the original drug chemically or enzymatically in the body after administration to exert their effects. For example, a composition for preventing or treating intestinal disease according to one embodiment of the present disclosure, comprising a host cell transformed with a recombinant vector containing a gene encoding an esterase represented by the nucleotide sequence of SEQ ID NO. 1 and trybutirin, can reach the intestine when administered orally as a prodrug. In this case, the esterase produced by the metabolism of the host cells established in the intestine can enhance the level of butyrate by hydrolyzing tributyrin. At this time, the desired level of butyrate in the intestine can be easily set by controlling tributyrin levels in the prodrug.

On the other hand, the prodrug consisting of the host cells and tributyltin can be formulated into liquid and solid pharmaceutical preparations, such as injectable solutions, suspensions, emulsions, tablets, coated tablets, coated tablet cores, capsules, solutions, troches, dispersions, pellets, granules, suppositories, hard or soft gelatin capsules, etc.

According to the features of the present disclosure, the prodrug composition for the prevention or treatment of intestinal diseases can be formulated in various compositions as long as it reaches the intestine, hydrolyzes tributyltin, and increases butyrate levels. For example, a prodrug composition for the prevention or treatment of intestinal diseases may comprise host cells transformed with a recombinant vector containing a gene indicated by the nucleotide sequence of SEQ ID NO. 1, wherein tributyltin may be included to improve butyrate production efficiency. Additionally, the prodrug composition for the prevention or treatment of intestinal diseases may comprise an esterase having at least 80% homology with the amino acid sequence of SEQ ID NO. 2 and tributyltin. At this time, the prodrug can be encapsulated to prevent degrading by gastric acid, antibiotics, bile acids, digestive enzymes, etc., until it reaches the intestine. Furthermore, the transformed strain can itself produce spores and may be a uniform strain that can germinate in the intestine.

Hereinafter, the present disclosure will be described in more detail through the embodiments. However, these embodiments are merely illustrative of the present disclosure and should not be interpreted as limiting the scope of the disclosure.

### The esterase-encoding gene according to an embodiment of the present disclosure and its use

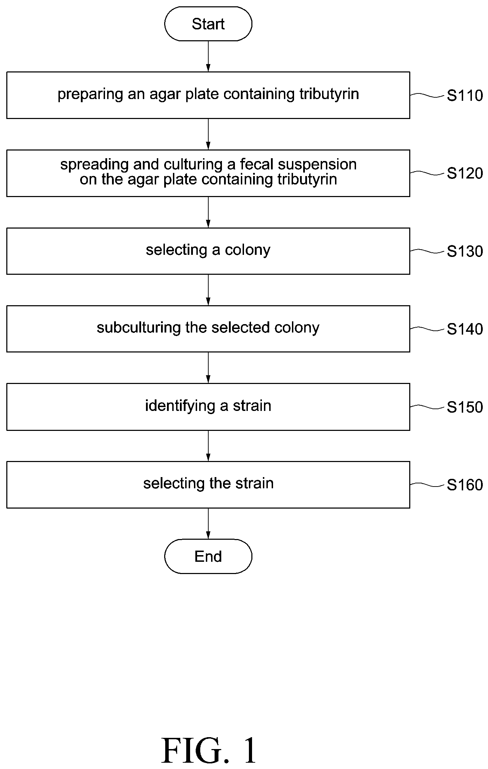

Hereinafter, with reference to Figs. 1 to 6, the esterase-encoding gene according to an embodiment of the present disclosure and its use will be described.

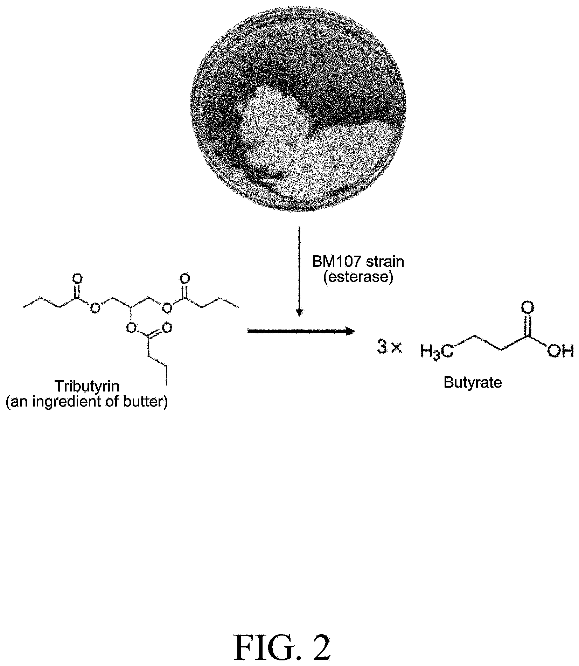

Fig. 1 is a flowchart of the identification process for Bacillus subtilis strains containing the esterase-encoding gene according to one embodiment of the present disclosure. At this time, for the convenience of explanation, reference will be made to Figs. 2 to 6.

The *Bacillus subtilis* strain containing the esterase-encoding gene, according to an embodiment of the present disclosure, may be specialized in producing butyrate by degrading tributyrin.

More specifically, referring to Fig. 1, the Bacillus subtilis strain containing the esterase-encoding gene according to an embodiment of the present disclosure is a strain identified by a process comprising preparing an agar plate containing tributyrin (S110); spreading and culturing a fecal suspension on the agar plate containing tributyrin (S120); selecting a colony (S130); subculturing the selected colony (S140); identifying a strain (S150); and selecting the strain (S160).

First, in preparing the agar plate (S110), the agar plate containing tributyrin comprises tributyrin at about 1%, and due to tributyrin, the agar plate appears visibly opaque.

Next, in the step of spreading and culture (S120), the fecal suspension may refer to a suspension in which fecal matter obtained from a human is suspended in a solvent to be dispersed therein, wherein the solvent may be a liquid medium or phosphate buffer (PBS) used in microorganism or bacterial culture experiments.

Furthermore, the fecal suspension may be a solution that has been primarily suspended and then secondarily dissolved and diluted. More specifically, the suspension that is primarily suspended may be a diluted solution that is serially diluted by 1/10 with the aforementioned solvent at a ratio of 1:9.

Next, in the spreading and culturing (S120), the fecal suspension prepared and diluted by the above-described process is spread onto an agar plate containing tributyrin at a concentration of about 1%, and the agar plate spread with the fecal suspension can be cultured for about 48 hours under conditions of 37°C.

Next, in the selecting colonies (S130), colonies with a clear zone formed around them within the agar plate after approximately 48 hours of culture can be selected. A clear zone can form as tributyrin in the agar plate is degraded by an esterase enzyme produced by bacteria in feces.

More specifically, referring to Fig. 2, a schematic diagram of the tributyrin-degrading process in Bacillus subtilis containing the esterase-encoding gene according to an embodiment of the present disclosure is shown. Tributyrin in the agar plate can be degraded into three butyrates by the bacterial esterase. Ultimately, when tributyrin is degraded by bacteria on an opaque agar plate containing tributyrin, a clear zone may form.

At this time, the bacteria that have esterase activity to degrade tributyrin, that is, bacteria that can produce esterase, may preferably be *Bacillus subtilis*, and the most preferred *Bacillus subtilis* used in an embodiment of the present disclosure is based on the Accession Number KCTC 15208 BP (BM107 strain), and may include bacteria with enhanced intrinsic activity for esterase due to a specific gene, namely, the esterase coding gene estB according to an embodiment of the present disclosure.

Ultimately, the bacteria selected through selecting a colony (S130) may be specialized strains that produce butyrate by degrading tributyrin.

Next, in the subculturing (S140), the selected colony can be subcultured on a new agar plate, depending on whether the clear zone is formed.

Then, in the identifying the strain (S150), the subcultured colonies can be identified by 16S rRNA sequencing, with *Bacillus subtilis*, *Lactobacillus sp*., and *Weissella sp*. identified.

Subsequently, in the selecting the strain (S160), strains were selected based on hemolytic activity. More specifically, the hemolytic activity of bacteria is a characteristic necessary for them to acquire the iron needed for survival within the host, and it can be considered an important toxic factor against the host in terms of its function. Therefore, since the strain must not exhibit this hemolytic activity to be utilized in the body, strains were selected based on the toxic factor associated with hemolytic activity, specifically β-hemolysin.

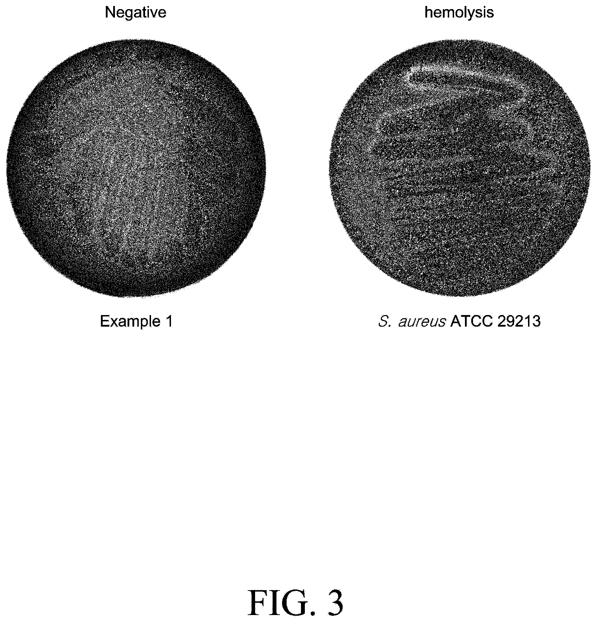

In this regard, Fig. 3 shows the results of hemolysis confirmation for Bacillus subtilis, a host cell containing the esterase-encoding gene according to one embodiment of the present disclosure. At this time, to confirm the hemolytic reaction, each strain was spread onto agar plate (Tryptic soy agar, TSA) containing 5 to 7% sheep blood and incubated at approximately 36 to 37 °C for about 24 hours, confirming hemolytic activity based on the presence of a hemolysis zone in the plate, that is, a clear zone, according to the activity of β-Hemolysin.

Referring to Fig. 3, the Example 1 of Bacillus subtilis containing the esterase-encoding gene according to one embodiment of the present disclosure shows no change in the medium.

In contrast, the control strain Staphylococcus aureus (*S. aureus*) showed the formation of a clear zone around the colony.

That is, the Example 1 of Bacillus subtilis containing the esterase-encoding gene according to one embodiment of the present disclosure indicates that the activity of β-Hemolysin is negative, meaning it does not cause hemolysis and is a stable strain. Therefore, as a host cell containing the esterase-encoding gene according to one embodiment of the present disclosure, Bacillus subtilis may be preferred, and thus, Example 1 was selected.

Furthermore, the selected Example 1 containing the esterase-encoding gene according to one embodiment of the present disclosure has been confirmed as a novel strain belonging to Bacillus subtilis and has been deposited at the Korea Research Institute of Bioscience and Biotechnology (KRIBB) with the Accession Number KCTC 15208 BP.

Referring back to Fig. 1, the strain selected in the selecting the strain (S160), which is the strain of Example 1 (deposited with the Accession Number KCTC 15208 BP), may exhibit activity of an esterase that can degrade tributyrin to butyrate as per the selecting a colony (S130).

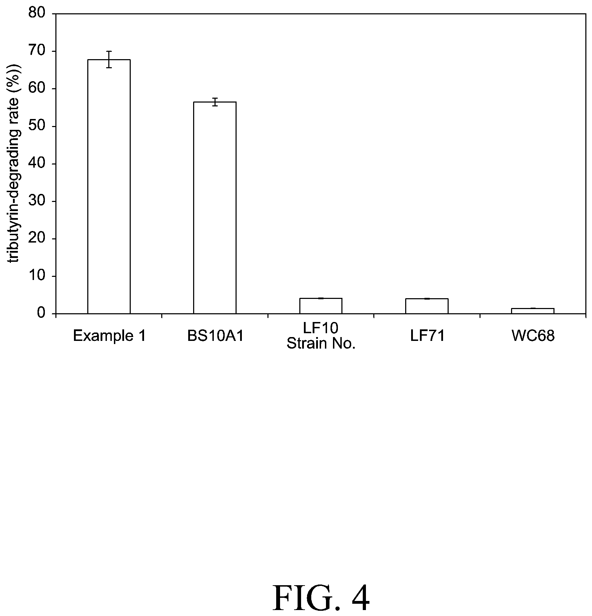

More specifically, referring to Fig. 4, the results of evaluating the tributyrin-degrading ability of the host cell containing the esterase-encoding gene according to one embodiment of the present disclosure are shown. At this time, based on the previous identification of strains using 16S rRNA gene sequencing, strains of *Bacillus subtilis, Lactobacillus sp., and Weissella sp.* were identified, and for the comparative evaluation of tributyrin-degrading ability for Example 1, standard strains BS10A1 (Bacillus subtilis), LF10 (Lactobacillus sp.), LF71 (Lactobacillus sp.), and WC68 (Weissella sp.) were used in *in vitro* comparative experiments. Furthermore, for *in vitro* experiments to evaluate tributyrin-degrading ability, each strain was inoculated into a liquid medium containing 10 mM tributyrin, and then a shaken culture was performed for about 24 hours at 37 °C and 200 rpm. Then, to measure the butyrate content in the culture medium, the culture medium was quantitatively analyzed by high-performance liquid chromatography (HPLC), and the amount of butyrate was compared with the theoretical maximum butyrate to evaluate the degrading rate per strain.

The degrading rate of tributyrin in Example 1 of Bacillus subtilis containing the esterase-encoding gene according to one embodiment of the present disclosure, was found to be approximately 69%, indicating a higher degrading rate than BS10A1 (Bacillus subtilis), LF10 (Lactobacillus sp.), LF71 (Lactobacillus sp.), and WC68 (Weissella sp.).

More specifically, the degrading rate of BS10A1, which is the same species as Example 1 containing the esterase-encoding gene according to one embodiment of the present disclosure, was approximately 56%, indicating that the degrading rate of Example 1 is more than 1.2 times higher.

Furthermore, the degrading rate of Lactobacillus strains LF10 and LF71 is approximately 4%, whereas the degrading rate of Example 1 is more than 17 times higher.

Additionally, the degrading rate of Weissella strain WC68 is approximately 2%, whereas that of Example 1 is more than 34.5 times higher.

In other words, among the identified strains, Example 1 does not exhibit toxic activity towards the host and may be a strain with enhanced esterase activity (production).

Ultimately, the host cell containing the esterase-encoding gene according to one embodiment of the present disclosure may include the Bacillus subtilis strain with Accession Number KCTC 15208 BP, which is Example 1.

Meanwhile, the host cell containing the esterase-encoding gene according to one embodiment of the present disclosure may include the Bacillus subtilis strain with Accession Number KCTC 15208 BP, which is Example 1, but is not limited to this, and may include various host cells and Bacillus subtilis strains containing specific genes derived from Example 1.

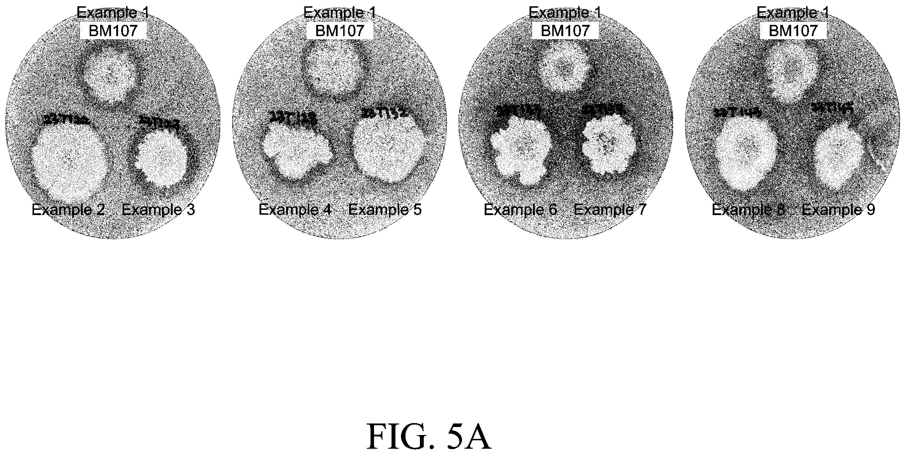

In this regard, referring to Fig. 5a, the results of strain selection based on the tributyrin-degrading ability of Bacillus subtilis containing the esterase-encoding gene according to one embodiment of the present disclosure are shown. At this time, for the evaluation of tributyrin-degrading ability of Bacillus subtilis according to one embodiment of the present disclosure, a total of 9 strains of Bacillus subtilis isolated from the feces of healthy individuals were used. Furthermore, for in vitro experiments to evaluate tributyrin degradation ability, a 1% BCP solution based on 10 ml of EtOH (absolute) and 0.1 g of BCP (Bromocresol purple) was added to TB-LB agar plates to prepare 0.01% TB-BCP agar plates, and then each strain was inoculated (spotted) at a dose of 10 µl with OD=1. At this time, each strain was inoculated at 2% into 2 mL of LB (LB + 1% TB supplement) and cultured overnight (18 hours). Furthermore, the strains inoculated with 10 µl each were cultured for 48 hours at 37°C and then observed.

The Bacillus subtilis strains containing the esterase-encoding gene according to one embodiment of the present disclosure, Examples 1 to 9, all showed a clear zone formed around the colonies on the plate. In other words, the Bacillus subtilis strains containing the esterase-encoding gene according to one embodiment of the present disclosure, Examples 1 to 9, can all be said to possess tributyrin-degrading ability.

Furthermore, tributyrin can be degraded by bacterial esterase activity, which can be expressed by specific genes. More specifically, Example 1 was confirmed to include the esterase-encoding gene estB (SEQ ID NO. 1), as indicated by esterase activity based on previous 16S rRNA sequence identification results, and its sequence is as follows: At this time, the amino acid composition of the estB protein (esterase) formed from the estB gene, indicated by SEQ ID NO. 1 according to one embodiment of the present disclosure, is the same as that of SEQ ID NO. 2 below, and the estB protein (esterase) primarily degrades neutral fats composed of short-chain fatty acids ranging from C3 to C10.
estB gene: 
GTGAAAAAAGTACTTATGGCATTCATTATTTGTTTATCGCTGATTCTATCTGTTTTAG
CCGCTCCGCCGTCTGGCGCAAAAGCTGAGTCAGTACATAATCCTGTCGTTCTTGTTC
ATGGAATAAGTGGTGCATCATACAACTTTTTCGCTATTAAAAACTACTTAATTTCTCA
AGGCTGGCAAAGCAACAAACTGTACGCAATTGATTTTTATGATAAAACAGGAAACA
 
ACCTAAATAACGGCCCGCAGCTTGCTTCATATGTTGACCGTGTTTTAAAAGAGACTG
GGGCAAAAAAAGTAGATATTGTGGCTCATAGTATGGGAGGCGCCAATACGCTGTAC
TATATTAAATATTTAGGCGGGGGCAATAAGATTCAAAATGTCGTAACGCTTGGAGG
GGCTAATGGTTTAGTGTCATCAACCGCGCTGCCGGGCACAGACCCTAATCAAAAGAT
CCTCTATACATCTATTTACAGTCTCAATGATCAAATTGTCATCAATAGCTTGTCTCGG
TTACAAGGAGCGCGAAACATCCAGCTTTATGGCATCGGTCATATTGGCTTGCTTTCT
AATAGCCAAGTGAACGGCTATATCAAAGAAGGGCTGAATGGCGGAGGCCTCAATAC
AAATTAA-3' (SEQ ID NO. 1)
estB protein:

MKKVLMAFIICLSLILSVLAAPPSGAKAESVHNPVVLVHGISGASYNFFAIKNYLISQGW
QSNKLYAIDFYDKTGNNLNNGPQLASYVDRVLKETGAKKVDIVAHSMGGANTLYYIKY
LGGGNKIQNVVTLGGANGLVSSTALPGTDPNQKILYTSIYSLNDQIVINSLSRLQGARNIQ
LYGIGHIGLLSNSQVNGYIKEGLNGGGLNTN (SEQ ID NO. 2)

Furthermore, it is shown that the estB gene indicated by the nucleotide sequence of SEQ ID NO. 1 includes not only Example 1 but also Examples 2 to 9, which have tributyltin degrading capability.

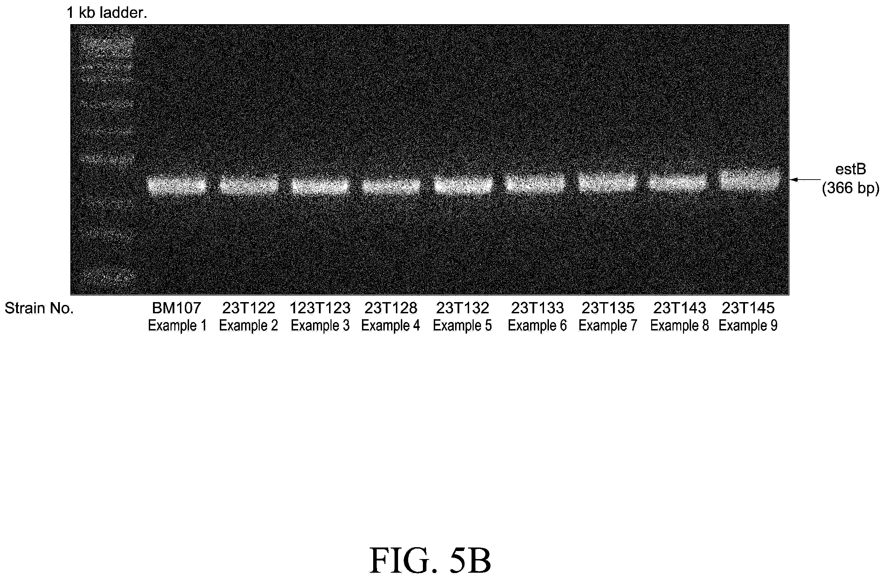

More specifically, referring to Fig. 5b, the PCR results of Bacillus subtilis containing the esterase-encoding gene according to one embodiment of the present disclosure are shown. At this time, in order to confirm whether the estB gene indicated by the nucleotide sequence of SEQ ID NO. 1 is present, a primer set based on the estB gene indicated by the nucleotide sequence of SEQ ID NO. 1 was used for SEQ ID NO. 3 (estB inner F: TCA AGG CTG GCA AAG CAA CA) and SEQ ID NO. 4 (estB inner R: TGG ATG TTT CGC GCT CCT TGT A). Furthermore, for the PCR experiment to confirm the estB gene indicated by the nucleotide sequence of SEQ ID NO. 1, a sample of 20 µl was prepared, which included 14.5 µl of D.W, 2 µl of 10X Ex Taq buffer, 1 µl of dNTP Mix, 0.5 µl of Ex Taq, 0.5 µl of primer forward, 0.5 µl of primer reverse, and 1 µl of gDNA, and the PCR conditions were set to 94.0 °C for 10 min, 94.0 °C for 30 sec, 60.0 °C for 30 sec, 72.0 °C for 50 sec, 72.0 °C for 5 min, and 4.0 °C, and the PCR was performed.

As a result of the PCR, it is shown that all Examples 1 to 9 have bands at 366 bp corresponding to the estB gene. That is, all Examples 1 to 9 of Bacillus subtilis containing the esterase-encoding gene according to one embodiment of the present disclosure include the estB gene indicated by the nucleotide sequence of SEQ ID NO. 1, and the expression of the estB gene indicated by the nucleotide sequence of SEQ ID NO. 1 may indicate that the esterase has activity due to the reduction in the expression.

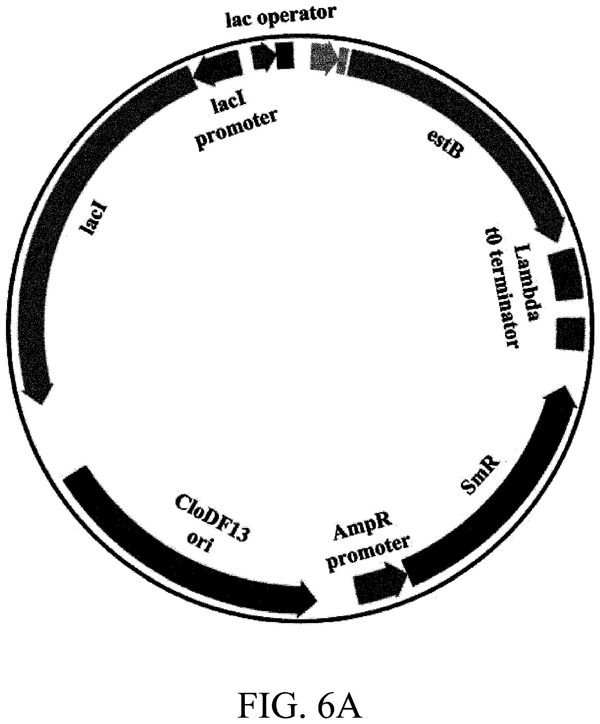

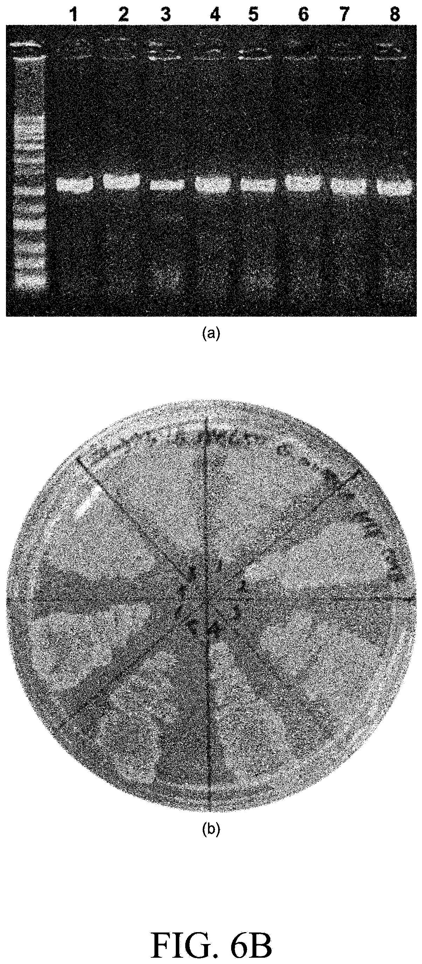

Furthermore, to confirm the enzyme's tributyltin-degrading capability for the estB gene, indicated by the nucleotide sequence of SEQ ID NO. 1, which contributes to butyrate production activity in Bacillus subtilis containing the esterase-encoding gene according to one embodiment of the present disclosure, a recombinant Escherichia coli strain was produced and verified. Thus, referring to Figs. 6a and 6b show, according to one embodiment of the present invention, the transformed Escherichia coli containing an esterase-coding gene, along with its tributyrin degradation ability.

First, Fig. 6a is a vector image containing the esterase-encoding gene (estB gene) indicated by the nucleotide sequence of SEQ ID NO. 1 according to one embodiment of the present disclosure, and referring to Fig. 6a, for the generation of recombinant strains, *E. coli* DH5α was used as the host, and the pCDF vector was used as the gene delivery vehicle. A pCDF vector for transformed Escherichia coli containing an esterase-coding gene according to one embodiment of the present disclosure originates from CloDF13 and includes a small multidrug resistance (SMR) gene for the active release mechanism in relation to resistance, serving as a selection marker, as a spectinomycin resistance gene, and includes J230106 as a promoter.

Next, GA_estB_F, GA_estB_R primers and PCR (annealing temperature 59°C, elongation time 20 seconds) were used to obtain an amplicon for the esterase-encoding gene (estB gene) indicated by the nucleotide sequence of SEQ ID NO. 1 according to one embodiment of the present disclosure, and then the backbone of the vector having CloDF13 and the spectinomycin resistance gene was amplified.

After gel purification of the amplified estB amplicon (insert) for the esterase-encoding gene indicated by the nucleotide sequence of SEQ ID NO. 1, according to one embodiment of the present disclosure, and the vector, the concentration was checked, and the mixture was prepared at a 3:1 ratio. The recombinant host used was *E. coli* DH5α, and the Gibson assembly was conducted at 50°C for 30 minutes using NEBuilder^{®} HiFi DNA Assembly Master Mix (Cat no: E2621S). At this time, the detailed conditions for the Gibson assembly are as shown in Table 1 below.

**[Table 1]**

| | |
|---|---|
| Vector(60ng) | 1.63µl |
| Insert(32.81ng) | 0.74µl |
| 2x Master mix(0.5x) | 2.5µl |
| D.W(up to 10µl) | 5.13µl |
| Total | 10µl |

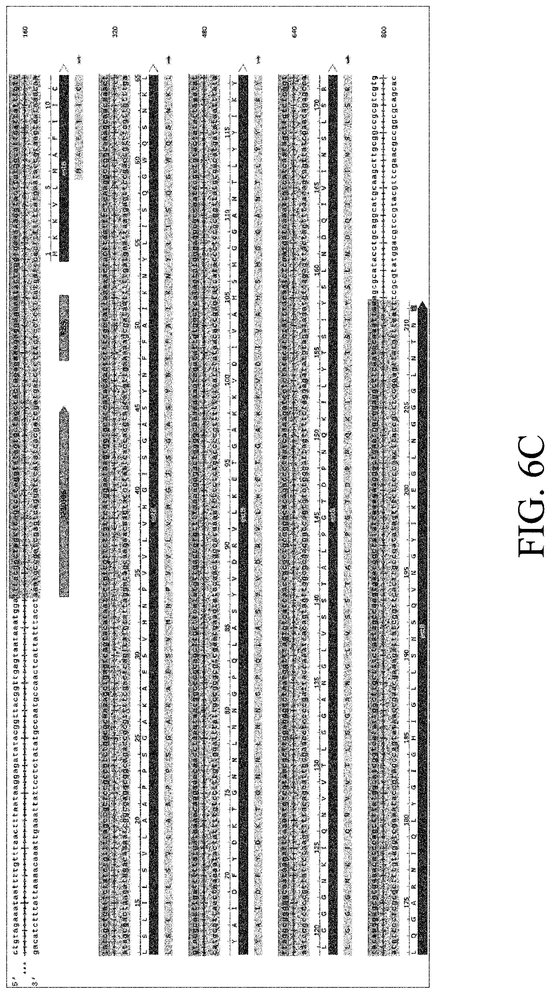

Fig. 6b shows the results of confirming whether an amplicon is produced for the esterase-coding gene (estB gene) indicated by the nucleotide sequence of SEQ ID NO. 1 according to one embodiment of the present invention in the recombinant E. coli (*E. coli* harboring pCDF estB) generated in the aforementioned Fig. 6a. At this time, to confirm amplicon generation, the cells were streaked onto LB agar plates (Spec50) containing spectinomycin (50 ug/ml) and incubated at 37°C for about 16 hours, after which eight typical colonies were randomly selected. Furthermore, Forward primer: PCR was performed using IVT_T7 and the Reverse primer pET21b_Terminator to confirm the presence of an amplicon for the esterase-encoding gene (estB gene) in the 1108 bp region, as indicated with SEQ ID NO. 1, according to one embodiment of the present disclosure. Referring to (a) of Fig. 6b, it shows that all randomly selected colonies numbered 1 to 8 contain the esterase-encoding gene (estB gene) indicated by SEQ ID NO. 1 according to one embodiment of the present disclosure. Furthermore, referring to (b) of Fig. 6b, colonies 1, 3, 5, and 7 were selected as spectinomycin-resistant colonies as they did not exhibit a light green color. Thus, referring to Fig. 6c, the results of Sanger sequencing analysis for colonies 1, 3, 5, and 7 confirmed in the aforementioned Fig. 6b are shown, indicating that colonies 3 and 7 among the recombinant E. coli generated by the aforementioned process contain the esterase-encoding gene (estB gene) indicated by SEQ ID NO. 1 according to one embodiment of the present disclosure.

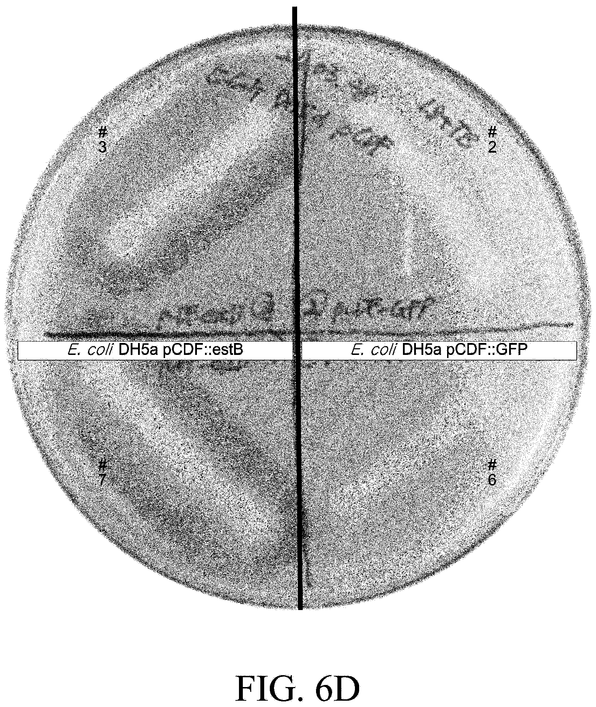

Furthermore, referring to Fig. 6d, to confirm the tributyrin-degrading ability of the transformed host cells, the results of confirming the clear zone for colonies of recombinant E. coli (*E. coli* harboring pCDF estB) No. 3 and 7, into which the esterase-coding gene (estB gene) indicated by the nucleotide sequence of SEQ ID NO. 1 according to one embodiment of the present invention selected in Fig. 6c is inserted are shown, at this time, a plate medium (LB+TB supplement 1%) containing 1% tributyrin (TB) was prepared, and then colonies of the aforementioned recombinant E. coli (#3, #7) and control E. coli (E. coli harboring pCDF GFP/#2, #6) were plated, and the tributyrin degradation ability was evaluated by confirming the clear zone formed around the plated area. Furthermore, the control E. coli (*E. coli* harboring pCDF GFP/#2, #6) is a recombinant E. coli containing a gene that includes a sequence with mutations in some regions of the esterase-encoding gene (estB gene) indicated by SEQ ID NO. 1 according to one embodiment of the present disclosure, allowing for a comparative confirmation of the functional effects on the intact esterase-encoding gene (estB gene) indicated by SEQ ID NO. 1 according to one embodiment of the present disclosure.

The E. coli (#3/#7) containing the esterase-encoding gene according to one embodiment of the present disclosure shows that a clear zone is formed in the surrounding area, while the control E. coli (#2/#6) shows no clear zone.

In other words, the introduction of the esterase-encoding gene (estB gene) indicated by SEQ ID NO. 1 according to one embodiment of the present disclosure into E. coli means that the esterase protein is expressed to degrade tributyrin and produce butyrate from it. Therefore, it can be suggested that the esterase-coding gene (estB) indicated by the nucleotide sequence of SEQ ID NO. 1, according to one embodiment of the present invention, enhances the intrinsic activity of esterase for the production of butyrate in various microorganisms.

Ultimately, the host cell containing the esterase-encoding gene, according to one embodiment of the present disclosure, can include not only the Example 1, strain deposited under Accession Number KCTC 15208 BP, but also all bacteria that contain the estB gene. For example, the host cell containing the esterase-encoding gene, according to one embodiment of the present disclosure, may include Bacillus subtilis or E. coli transformed to contain the estB gene, but is not limited to this.

According to the above process, Bacillus subtilis, according to one embodiment of the present disclosure, which has enhanced butyrate production activity by degrading tributyrin, can be selected, and furthermore, an esterase-encoding gene indicated by SEQ ID NO. 1 derived from Bacillus subtilis that can enhance the intrinsic activity of esterases can be derived.

Furthermore, the selected Bacillus subtilis containing the esterase-encoding gene according to one embodiment of the present disclosure is a bacterium that has secured safety without hemolytic activity in the host and can be used in the host's body as food or medicine.

For example, the host cell Bacillus subtilis containing the esterase-encoding gene, according to one embodiment of the present disclosure, can be used to provide a prodrug composition for the prevention or treatment of inflammatory bowel disease. More specifically, a prodrug is a drug that has been chemically modified to adjust its physical and chemical properties, which does not exhibit physiological activity in itself but can be converted into the original drug by chemical means or by enzyme action after administration to exert its therapeutic effect. Thus, the host cell Bacillus subtilis containing the esterase-encoding gene, according to one embodiment of the present disclosure, can be used as a prodrug composition for the prevention or treatment of inflammatory bowel disease, and may include the Bacillus subtilis containing the esterase-encoding gene according to the aforementioned embodiment of the present disclosure.

On the other hand, the Bacillus subtilis containing the esterase-encoding gene according to one embodiment of the present disclosure ultimately aims to produce butyrate from tributyrin, so the prodrug composition for the prevention or treatment of inflammatory bowel disease containing Bacillus subtilis containing the esterase-encoding gene according to one embodiment of the present disclosure may further include tributyrin to enhance butyrate production efficiency along with the bacteria.

Furthermore, the selected host cell, Bacillus subtilis, containing the esterase-encoding gene according to one embodiment of the present disclosure, due to the inclusion of the estB gene, activates the esterase, thereby improving butyrate production efficiency. Moreover, if enhanced butyrate production occurs in the host's body, the selected host cell, Bacillus subtilis, containing the esterase-encoding gene according to one embodiment of the present disclosure, can treat or prevent the host's intestinal diseases. According to one embodiment of the present disclosure, the host cell containing the esterase-encoding gene is not limited to Bacillus subtilis and can include any host cell containing the estB gene. For example, the transformed Escherichia coli to contain the estB gene, as indicated by SEQ ID NO. 1, can also have the same effect.

### Verification of the esterase-encoding gene and the host cell containing it according to one embodiment of the present disclosure

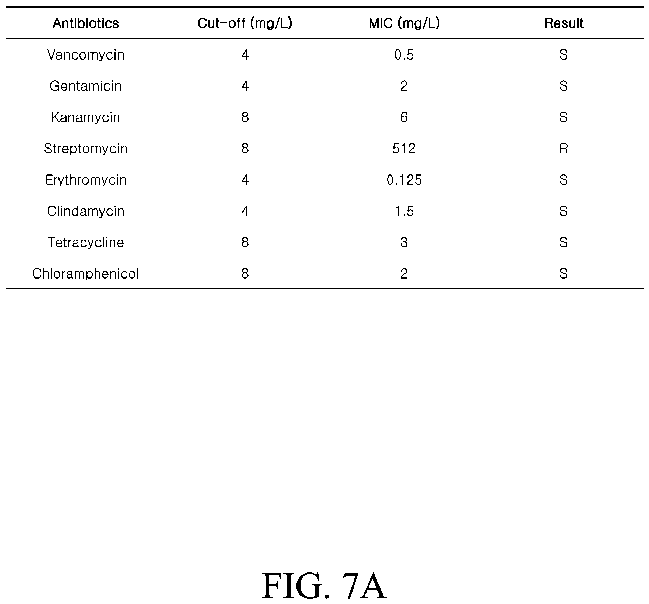

Hereinafter, with reference to Figs. 7a to 8c, the antibiotic resistance and therapeutic efficacy of the host cell containing the esterase-encoding gene according to one embodiment of the present disclosure will be verified.

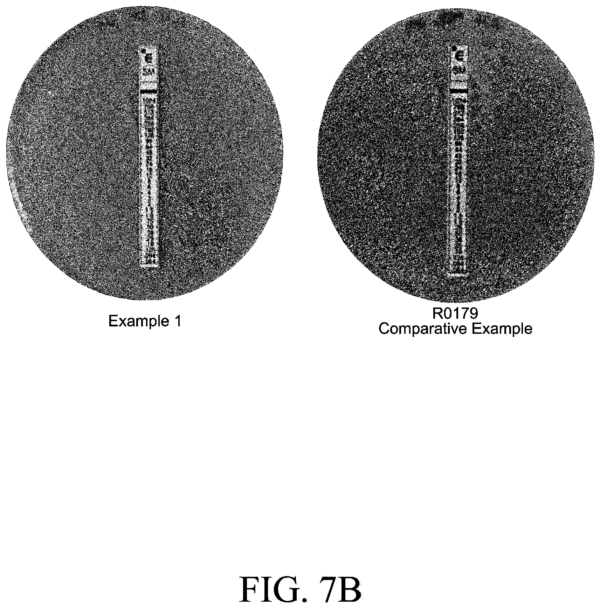

Figs. 7a and 7b show the experimental results regarding the antibiotic resistance of the host cell containing the esterase-encoding gene according to one embodiment of the present disclosure according to one embodiment of the present disclosure. At this time, the antibiotic susceptibility test was performed using the E-TEST strip (bioMerieux). More specifically, as the host cell containing the esterase-encoding gene according to one embodiment of the present disclosure according to one embodiment of the present disclosure, Example 1, which is strain Bacillus subtilis deposited under the Accession Number KCTC 15208 BP, was cultured overnight, and the cultured strain of Example 1 was diluted with 1X PBS to match the 0.5 McFarland standard. The diluted strain was spread on Tryptic Soy Agar medium, and after placing the E-test strip on the spread medium, it was incubated under anaerobic conditions for 48 hours. Furthermore, the antibiotic cut-off value was based on EFSA (European Food Safety Authority) standards.

First, referring to Fig. 7a, the MIC concentration of Example 1, strain according to one embodiment of the present disclosure, is shown to be below the cut-off value for seven antibiotics (vancomycin, gentamicin, kanamycin, erythromycin, clindamycin, tetracycline, chloramphenicol), excluding Streptomycin. That is, it can be inferred that Bacillus subtilis, which is the host cell containing the esterase-encoding gene according to one embodiment of the present disclosure, lacks antibiotic resistance.

Meanwhile, for the antibiotic Streptomycin, the MIC concentration of Example 1 is found to exceed the cut-off value.

However, this appears to be at a level similar to that of commercially available pharmaceutical strains. More specifically, referring to Fig. 7b, the results of the resistance test for Streptomycin against the strain B. subtilis R0179 (comparative example) isolated from the commercially available pharmaceutical Medilac-DS are shown, and the MIC of Example 1 according to one embodiment of the present disclosure is shown to form a clear zone (indicated by an oval) similar to that of the comparative example. That is, it can be inferred that Bacillus subtilis, which is the host cell containing the esterase-encoding gene according to one embodiment of the present disclosure, has a resistance similar to that of strains approved and sold in the market for the antibiotic Streptomycin.

Therefore, Bacillus subtilis, which is the host cell containing the esterase-encoding gene according to one embodiment of the present disclosure, and the composition containing it may not cause antibiotic resistance, and thus may be safe from risks such as treatment failure due to antibiotic resistance, loss of therapeutic selectivity, and increased likelihood and severity of disease.

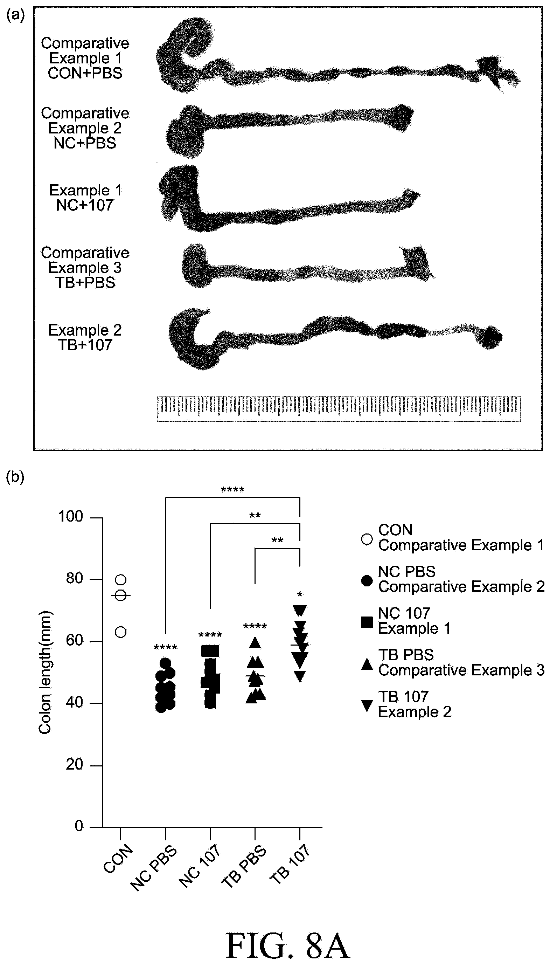

Figs. 8a to 8c show the results of verifying the therapeutic efficacy against inflammatory bowel disease (IBD) in a host cell, Bacillus subtilis, containing an esterase-coding gene according to one embodiment of the present invention, and a prodrug composition for the prevention or treatment of inflammatory bowel disease containing the same. At this time, male C57BL/6 mice aged 9 weeks were used to verify therapeutic efficacy against IBD. More specifically, the therapeutic efficacy verification experiment was conducted after a one-week adaptation period in 9-week-old mice, by administering 2% DSS (dextran sodium sulfate) in drinking water for 5 days to induce IBD, and then replacing it with 1% DSS for another 5 days. Furthermore, after the DSS drinking water administration, regular drinking water without DSS was provided for 5 days. Comparative Example 1 (control + PBS), Comparative Example 2 (NC + PBS), and Example 1 (NC + strain deposited under the Accession Number KCTC 15208 BP according to one embodiment of the present disclosure) were fed a normal chow diet (NC), while Comparative Example 3 (TB + PBS) and Example 2 (TB + strain deposited under the Accession Number KCTC 15208 BP according to one embodiment of the present disclosure) were fed a normal diet for 5 days, and thereafter, from the 6th to the 9th day, a diet containing tributyrin (tributyrin diet, TB) was administered. Furthermore, Comparative Example 1 is a normal individual that does not include IBD, and the drinking water did not contain DSS. PBS and strains from Comparative Examples 1 to 3, Example 1, and Example 2 were treated by oral administration at a dose of 100 µl/day. The aforementioned experiments on mice were conducted in accordance with the Yonsei Biomedical Research Institute and regulations within the ARRIVE (Animal Research: Reporting of In Vivo Experiment) guidelines.

First, Fig. 8a shows the intestinal length results after treatment with Bacillus subtilis, a host cell containing an esterase-coding gene according to one embodiment of the present invention, and a prodrug composition for the prevention or treatment of inflammatory bowel disease containing the same.

Referring to (a) of Fig. 8a, the experimental groups with inflammatory bowel disease, namely Comparative Example 2, Example 1, Comparative Example 3, and Example 2, showed shorter colon lengths than the control group, Comparative Example 1, which did not develop inflammatory bowel disease. In other words, the length of the colon, which includes the colon, may be reduced due to inflammatory bowel disease.

However, referring to (b) of Fig. 8a, the colon length of Example 2 according to one embodiment of the present disclosure is approximately 50 to 70 mm, which is similar to the approximately 60 to 80 mm of the control group, Comparative Example 1, that does not include inflammatory bowel disease (IBD).

That is, it may mean that by treating a prodrug composition for the prevention or treatment of inflammatory bowel disease comprising Bacillus subtilis, a host cell containing an esterase-coding gene, and tributyrin according to one embodiment of the present invention, the inflammatory bowel disease can be restored to a level similar to that of a normal individual.

Furthermore, the colon length of Example 1, which is a composition solely containing the host cell Bacillus subtilis with an esterase-encoding gene according to one embodiment of the present disclosure, is longer than that of Comparative Example 2, which is an experimental group with intestinal disease that did not receive the strain. That is, it may mean that inflammatory bowel disease can be recovered even with the sole treatment of Bacillus subtilis, a host cell containing an esterase-coding gene, according to one embodiment of the present invention.

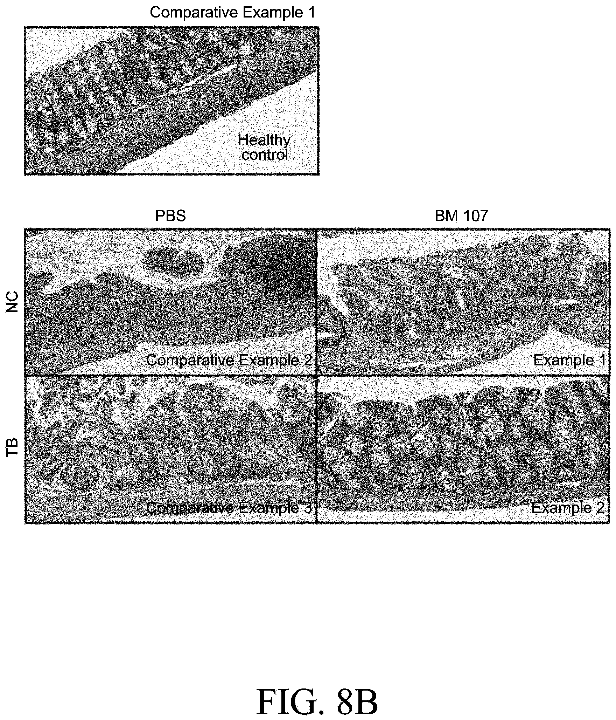

Further, referring to Fig. 8b, H&E stained images of colon tissues are illustrated, indicating that the colon tissues (epithelial cells) of Examples 1 and 2 have histological features similar to those of the healthy control group (Comparative Example 1), which does not include IBD. That is, as described above in Fig. 8b, treatment with a host cell, Bacillus subtilis, containing an esterase-coding gene according to one embodiment of the present invention, or a prodrug composition containing the same, may mean that inflammatory bowel disease can be cured.

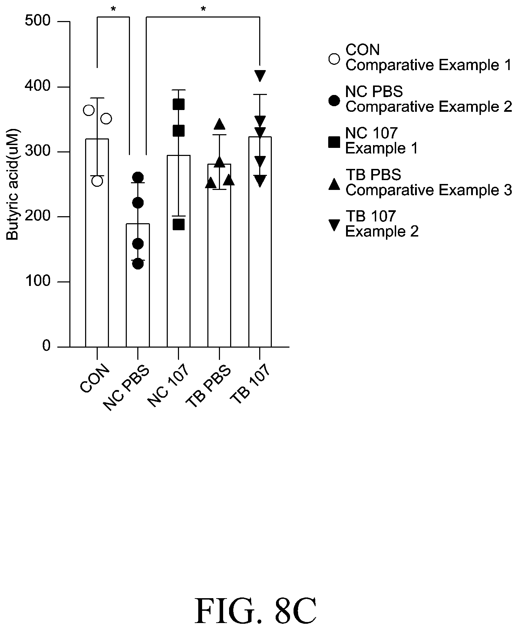

Furthermore, referring to Fig. 8c shows the results of intestinal butyrate concentrations after treatment with Bacillus subtilis, a host cell containing an esterase-coding gene according to one embodiment of the present invention, and a prodrug composition for the prevention or treatment of inflammatory bowel disease containing the same. At this time, the sample collection for measuring intestinal butyrate concentration was performed by first excising the cecum from the mouse, recovering the contents of the cecum, weighing the contents, adding 1 ml of PBS per 100 mg of cecal contents, and then centrifuging at 8,000 rpm for 15 minutes to obtain the supernatant, methanol equivalent to 4 times the volume of the obtained supernatant was added, and the mixture was again centrifuged at 8,000 rpm for 15 minutes to obtain the final analysis sample (supernatant). The butyrate of the obtained final analysis sample was measured using LC-MS (Liquid chromatography - mass spectrometry/Vanquish Flex UHPLC/Thermo Fisher Scientific Inc.). At this time, the column used was a Waters Cortects C8 column (2.1x150, 1.6µm), and the column temperature was set to 50 °C. Furthermore, 2 mM Ammonium acetate and MeOH were used as the mobile phase for LC-MS.

The butyrate concentration in Comparative Example 2, Example 1, and Comparative Example 3, which include inflammatory bowel disease, is lower than that of the normal control group (Comparative Example 1). That is, the butyrate concentration in the cecum may decrease due to inflammatory bowel disease.

However, the butyrate concentration in Example 2, according to one embodiment of the present disclosure, is about 330 µm, which is higher than the approximately 320 µm in the control group of Comparative Example 1, which does not include IBD. This means that by treating a prodrug composition for the prevention or treatment of inflammatory bowel disease, which includes a host cell, Bacillus subtilis, containing an esterase-encoding gene according to one embodiment of the present disclosure and tributyrin, the butyrate level in the intestine can be restored to normal levels.

Furthermore, the butyrate concentration of the composition of Example 1, which includes only the host cell, Bacillus subtilis, containing an esterase-encoding gene according to one embodiment of the present disclosure, is found to be about 300 µm, which is higher than the approximately 195 µm of the experimental group with intestinal disease that did not receive the strain in Comparative Example 2. That is, it may mean that intestinal butyrate levels can be restored to normal individual levels even with the sole treatment of Bacillus subtilis, a host cell containing an esterase-coding gene, according to one embodiment of the present invention.

That is, Bacillus subtilis, the host cell containing an esterase-coding gene according to one embodiment of the present invention, and the prodrug composition for the prevention or treatment of inflammatory bowel disease containing the same can induce the active production of butyrate in the colon, and various functional effects such as alleviating inflammation of colon epithelial cells, strengthening the function of the intestinal barrier, suppressing colon polyps, and improving cognitive function can occur through the generated butyrate.

Meanwhile, as mentioned above, the host cell containing an esterase-coding gene according to one embodiment of the present invention, and the prodrug composition for the prevention or treatment of inflammatory bowel disease containing the same, may require a formulation and administration method that can reach the colon, depending on the intended activity in the host's intestine. For example, the host cell containing an esterase-coding gene according to one embodiment of the present invention, and the prodrug composition for the prevention or treatment of inflammatory bowel disease containing the same, can be formulated in capsule form, but is not limited to this and can also be formulated in rectally administrated form or orally ingestible form.

According to the above results, the host cell containing an esterase-coding gene according to one embodiment of the present invention, and the prodrug composition for the prevention or treatment of inflammatory bowel disease containing the same, can provide a high concentration of butyrate in the host's intestine, thereby treating or preventing various intestinal diseases, including inflammatory bowel disease.

At this time, the high concentration of butyrate can be attributed to the high esterase activity of Bacillus subtilis according to one embodiment of the present disclosure. Furthermore, the esterase activity that maximizes the degradation efficiency of tributyrin can be determined by the presence of a specific gene, namely, the estB gene, indicated by the nucleotide sequence of SEQ ID NO. 1, according to one embodiment of the present disclosure.

While the embodiments of the present disclosure have been described in more detail with reference to the attached drawings, the present disclosure is not necessarily limited to these embodiments, and various modifications can be implemented within the scope of the technical concept of the invention. Therefore, the embodiments disclosed in the invention are intended to describe, not to limit, the technical idea of the invention, and the technical scope of the invention is not restricted by these embodiments. Therefore, the embodiments described above should be understood as illustrative in all respects and not limiting. The scope of protection of this disclosure shall be interpreted according to the following claims below, and all technical concepts falling within the equivalent scope thereof shall be interpreted as being included in the scope of rights of this disclosure.

[Accession Number]
   Depositary Authority Name: Korean Collection for Type Cultures (KCTC)
   Accession Number: KCTC15208BP
   Accession Date: 20221128
[National R&D Project that supported this invention]
[Project ID] 1425177671
[Project No.] S3287890
[Ministry] Ministry of SMEs and Startups
[Project Management (Specialized) Agency] Korea Technology and Information Promotion Agency for SMEs
[Project Name] SME Technology Development Support Project
[Project Title] Development of Microbiome-based Microbial Therapeutics
[Executing Organization] BioMe Inc.
[Project Period] 2022.05.01 ~ 2024.04.30
[Project ID] 1711200956
[Project No.] 2022M3A9F3017506
[Ministry] Ministry of Science and ICT
[Project Management (Specialized) Agency] National Research Foundation of Korea
[Project Name] Development of Biomedical Technology
[Project Title] Construction and Validation of a Biodelivery System for Gene Delivery with Therapeutic Efficacy via CRISPR-based Genome Editing Technology
[Executing Organization] Yonsei University
[Project Period] April 1, 2022 ~ December 31, 2026

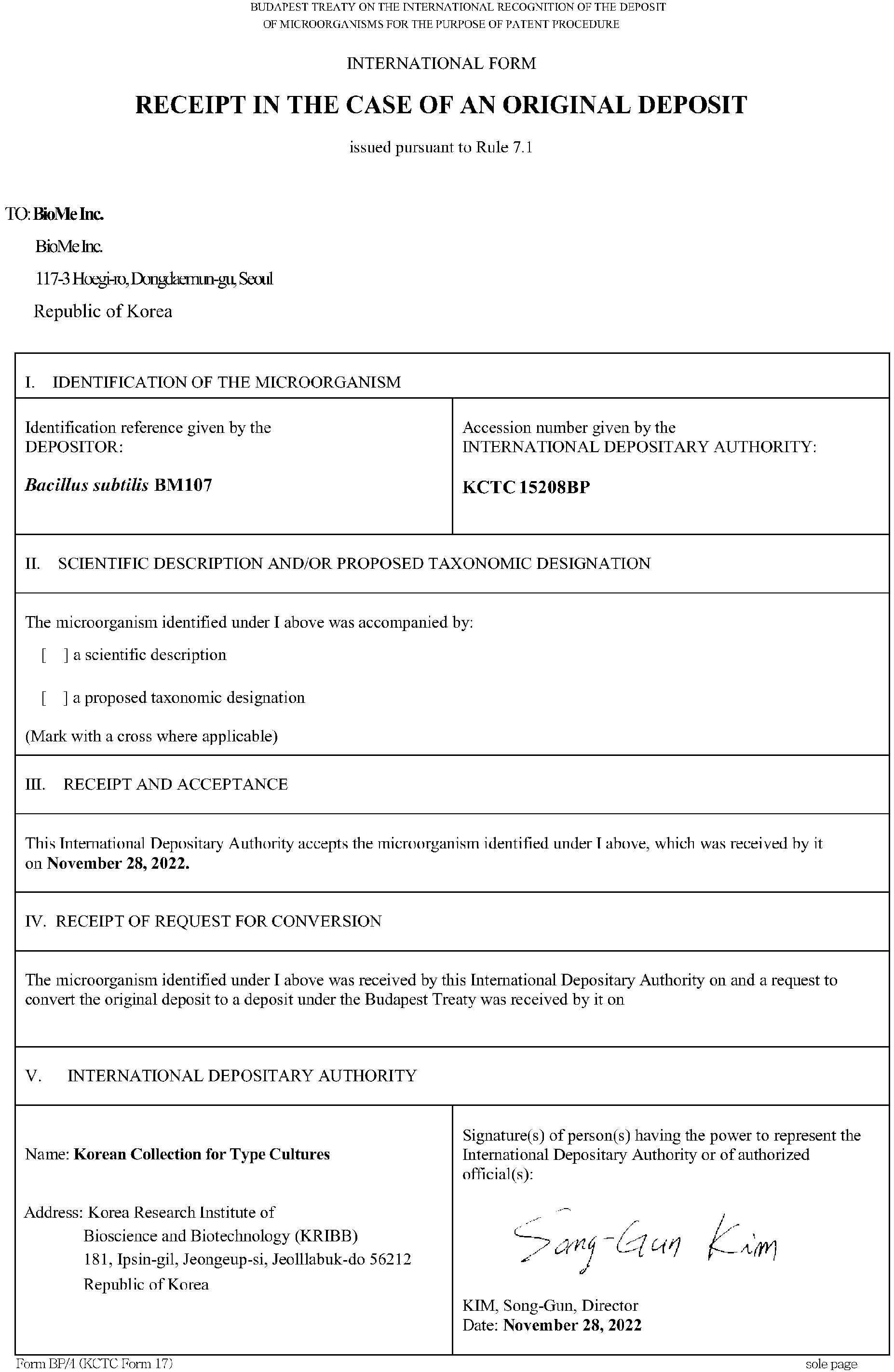
BUDAPEST TREATY ON THE INTERNATIONAL RECOGNITION OF THE DEPOSIT
OF MICROORGANISMS FOR THE PURPOSE OF PATENT PROCEDURE

INTERNATIONAL FORM

RECEIPT IN THE CASE OF AN ORIGINAL DEPOSIT

issued pursuant to Rule 7.1

TO: BioMe Inc.
BioMe Inc.
117-3 Hoegi-ro, Dongdaemun-gu, Seoul
Republic of Korea

I. IDENTIFICATION OF THE MICROORGANISM
Identification reference given by the
DEPOSITOR:

Bacillus subtilis BM107 | Accession number given by the
INTERNATIONAL DEPOSITARY AUTHORITY:

KCTC 15208BP
II. SCIENTIFIC DESCRIPTION AND/OR PROPOSED TAXONOMIC DESIGNATION
The microorganism identified under I above was accompanied by:
[ ] a scientific description
[ ] a proposed taxonomic designation
(Mark with a cross where applicable)
III. RECEIPT AND ACCEPTANCE
This International Depositary Authority accepts the microorganism identified under I above, which was received by it
on November 28, 2022.
IV. RECEIPT OF REQUEST FOR CONVERSION
The microorganism identified under I above was received by this International Depositary Authority on and a request to
convert the original deposit to a deposit under the Budapest Treaty was received by it on
V. INTERNATIONAL DEPOSITARY AUTHORITY
Name: Korean Collection for Type Cultures

Address: Korea Research Institute of
Bioscience and Biotechnology (KRIBB)
181, Ipsin-gil, Jeongeup-si, Jeolllabuk-do 56212
Republic of Korea | Signature(s) of person(s) having the power to represent the
International Depositary Authority or of authorized
official(s):


KIM, Song-Gun, Director
Date: November 28, 2022

Form BP/4 (KCTC Form 17) sole page

## Claims

1. A gene indicated by the nucleotide sequence of SEQ ID NO. 1 encoding for esterase derived from *Bacillus subtilis*.

2. An esterase indicated by the amino acid sequence of SEQ ID NO. 2.

3. The esterase according to claim 2,
wherein the esterase produces butyrate by degrading tributyrin.

4. The esterase according to claim 2,
wherein the esterase expressed from a gene indicated by the nucleotide sequence of SEQ ID NO. 1 derived from *Bacillus subtilis*.

5. A recombinant vector containing the gene encoding the esterase of claim 1.

6. A host cell transformed with the recombinant vector of claim 5.

7. The host cell according to claim 6,
wherein the host cells is **characterized by** producing an esterase indicated by the amino acid sequence of SEQ ID NO. 2.

8. The host cell according to claim 7,
wherein the host cell is **characterized by** having increased activity of the esterase compared to the wild type.

9. The host cell according to claim 6,
wherein the host cell is *Escherichia coli* or *Bacillus subtilis*.

10. *Bacillus subtilis* BM107, deposited under accession number KCTC 15208BP, with excellent butyrate production capacity.

11. The *Bacillus subtilis* BM107 according to claim 10,
wherein the Bacillus subtilis BM107 is an overexpressed gene indicated by the nucleotide sequence of SEQ ID NO. 1.

12. The *Bacillus subtilis* BM107 according to claim 10,
wherein the Bacillus subtilis BM107 is **characterized by** producing butyrate by degrading tributyrin.

13. The *Bacillus subtilis* BM107 according to claim 10,
wherein the Bacillus subtilis BM107 has negative activity of β-Hemolysin.

14. The *Bacillus subtilis* BM107 according to claim 10,
wherein the Bacillus subtilis BM107 does not have resistance to at least one of the antibiotics vancomycin, gentamicin, kanamycin, erythromycin, clindamycin, tetracycline, chloramphenicol.

15. The *Bacillus subtilis* BM107 according to claim 10,
wherein the Bacillus subtilis BM107 has a tributyrin-degrading rate more than 1.2 times that of Bacillus subtilis lacking the gene indicated by the nucleotide sequence of SEQ ID NO. 1.

16. The *Bacillus subtilis* BM107 according to claim 10,
wherein the Bacillus subtilis BM107 has a tributyrin-degrading rate more than 17 times that of Lactobacillus sp.

17. The *Bacillus subtilis* BM107 according to claim 10,
wherein the Bacillus subtilis BM107 has a tributyrin-degrading rate more than 34.5 times that of Weissella sp.

18. A pharmaceutical composition for the prevention or treatment of inflammatory bowel disease, containing host cells of any one of claims 6 to 9 and tributyrin.

19. The pharmaceutical composition according to claim 18,
wherein the inflammatory bowel disease comprises at least one of ulcerative colitis, Crohn's disease, collagenous colitis, lymphocytic colitis, ischemic colitis, transitional colitis, and Behçet's syndrome.

20. A method for enhancing esterase activity comprising,
transforming host cells with a recombinant vector containing the gene indicated by the nucleotide sequence of SEQ ID NO. 1, and
overexpressing the gene indicated by the nucleotide sequence of the above SEQ ID NO. 1,
wherein the gene indicated by the nucleotide sequence of the above SEQ ID NO. 1 is a gene encoding an esterase indicated by the amino acid sequence of SEQ ID NO. 2.
